# EUROPEAN PATENT SPECIFICATION

(11) **EP 1 877 364 B1**
(45) Date of publication and mention of the grant of the patent: **08.04.2009**
(21) Application number: 06724538.1
(22) Date of filing: 24.04.2006
(51) Int. Cl.: C07C 211/52, C07D 277/42, C07D 231/38, C07D 213/74, C07D 241/42, C07D 241/44, C07D 215/40, C07D 487/04, C07D 231/56, C07D 285/14, C07D 405/04, C07D 239/12, C07D 241/20, C07D 233/54, C07D 235/06

(54) **PHENYLACETYLENE DERIVATIVES HAVING MGLUR5 RECEPTOR AFFINITY**
PHENYLACETYLENDERIVATE MIT AFFINITÄT ZUM MGLUR5-REZEPTOR
DERIVES DE PHENYLACETYLENE POSSEDANT UNE AFFINITE POUR LE RECEPTEUR MGLUR5

(30) Priority: 25.04.2005 GB 0508318
(43) Date of publication of application: 16.01.2008
(73) Proprietor: NOVARTIS AG, 4002 Basel (CH); Novartis Pharma GmbH, 1230 Vienna (AT)
(72) Inventor: GLATTHAR, Ralf, 79713 Bad Säckingen (DE); TROXLER, Thomas, J., CH-4246 Wahlen b. Laufen (CH)
(74) Representative: Vögeli-Lange, Regina
(86) International application number: PCT/EP2006/003764
(87) International publication number: WO 2006/114260

(56) References cited:
- WO-A-03/047581
- CHUA ET AL: "Cyclohexenyl- and dehydropiperidinyl-alkynyl pyridines as potent metabotropic glutamate subtype 5 (mGlu5) receptor antagonists" BIOORGANIC & MEDICINAL CHEMISTRY LETTERS, OXFORD, GB, vol. 15, no. 20, 15 October 2005 (2005-10-15), pages 4589-4593, XP005064658 ISSN: 0960-894X

## Description

The present invention relates to novel acetylene derivatives, their preparation, their use as pharmaceuticals and pharmaceutical compositions containing them.

Certain acetylene derivatives and their pharmaceutical use are described in WO 03/047581.

More particularly the invention provides a compound of formula (I) 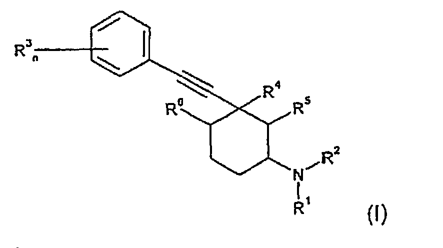 wherein
- R¹: represents hydrogen or C₁-C₄ alkyl and
- R²: represents an unsubstituted or substituted heterocycle or

- R¹: represents hydrogen or C₁-C₄ alkyl and
- R²: represents aryl or substituted aryl or

- R¹: and R² together with the nitrogen atom form an unsubstituted or substituted heterocycle
- R³: represents (C₁₋₄)alkyl, (C₁₋₄)alkoxy, trifluoromethyl, halogen, cyano, nitro, -CHO, -COO(C₁₋₄)alkyl, -CO(C₁₋₄)alkyl;
- n: represents 0, 1, 2, 3, 4 or 5;

- R⁴: represents OH and
- R⁵: and R⁶ represent H or C₁-C₄ alkyl or

- R⁴: and R⁵ form a bond and
- R⁶: represent H or C₁-C₄ alkyl or

- R⁴: and R⁶ form a bond and
- R⁵: represent H or C₁-C₄ alkyl;
in free base or acid addition salt form.

In the present specification, the following definitions shall apply if no specific other definition is given:

"Alkyl" represents a straight-chain or branched-chain alkyl group, preferably represents a straight-chain or branched-chain C₁₋₁₂alkyl, particularly preferably represents a straight-chain or branched-chain C₁₋₆alkyl; for example, methyl, ethyl, n- or iso-propyl, n-, iso-, sec- or tert-butyl, n-pentyl, n-hexyl, n-heptyl, n-octyl, n-nonyl, n-decyl, n-undecyl, n-dodecyl, with particular preference given to methyl, ethyl, n-propyl and iso-propyl.

"Alkandiyl" represents a straight-chain or branched-chain alkandiyl group bound by two different Carbon atoms to the molecule, it preferably represents a straight-chain or branched-chain C₁₋₁₂ alkandiyl, particularly preferably represents a straight-chain or branched-chain C₁₋₆ alkandiyl; for example, methandiyl (-CH₂-), 1,2-ethanediyl (-CH₂-CH₂-), 1,1-ethanediyl ((-CH(CH₃)-), 1,1-, 1,2-, 1,3-propanediyl and 1,1-, 1,2-, 1,3-, 1,4-butanediyl, with particular preference given to methandiyl, 1,1-ethanediyl, 1,2-ethanediyl, 1,3-propanediyl, 1,4-butanediyl.

Each alkyl part of "alkoxy", "alkoxyalkyl", "alkoxycarbonyl", "alkoxycarbonylalkyl" and "halogenalkyl" shall have the same meaning as described in the above-mentioned definition of "alkyl".

"Alkenyl" represents a straight-chain or branched-chain alkenyl group, preferably C₂₋₆ alkenyl, for example, vinyl, allyl, 1-propenyl, isopropenyl, 2-butenyl, 2-pentenyl, 2-hexenyl, etc. and preferably represents C₂₋₄ alkenyl.

"Alkendiyl" represents a straight-chain or branched-chain alkendiyl group bound by two different Carbon atoms to the molecule, it preferably represents a straight-chain or branched-chain C₂₋₆ alkandiyl; for example, -CH=CH-, -CH=C(CH₃)-, -CH=CH-CH₂-, -C(CH₃)=CH-CH₂-,-CH=C(CH₃)-CH₂-, -CH=CH-C(CH₃)H-, -CH=CH-CH=CH-, -C(CH₃)=CH-CH=CH-, -CH=C(CH₃)-CH=CH-, with particular preference given to -CH=CH-CH₂-, -CH=CH-CH=CH-.

"Alkynyl" represents a straight-chain or branched-chain alkynyl group, preferably C₂₋₆ alkynyl, for example, ethenyl, propargyl, 1-propynyl, isopropenyl, 1- (2- or 3) butynyl, 1- (2- or 3) pentenyl, 1- (2- or 3) hexenyl, etc., preferably represents C₂₋₄alkynyl and particularly preferably represents ethynyl.

"Aryl" represents an aromatic hydrocarbon group, preferably a C₆₋₁₀ aromatic hydrocarbon group; for example phenyl, naphthyl, especially phenyl.

"Aralkyl" denotes an "Aryl" bound to an "Alkyl" (both as defined above) an represents, for example benzyl, α-methylbenzyl, 2-phenylethyl, α,α-dimethylbenzyl, especially benzyl.

"Heterocycle" represents a saturated, partly saturated or aromatic ring system containing at least one hetero atom. Preferably, heterocycles consist of 3 to 11 ring atoms of which 1-3 ring atoms are hetero atoms. Heterocycles may be present as a single ring system or as bicyclic or tricyclic ring systems; preferably as single ring system or as benz-annelated ring system. Bicyclic or tricyclic ring systems may be formed by annelation of two or more rings, by a bridging atom, e.g. Oxygen, sulfur, nitrogen or by a bridging group, e.g. alkandediyl or alkenediyl. A Heterocycle may be substituted by one or more substituents selected from the group consisting of Oxo (=O), Halogen, Nitro, Cyano, Alkyl, Alkandiyl, Alkenediyl, Alkoxy, Alkoxyalkyl, Alkoxycarbonyl, Alkoxycarbonylalkyl, Halogenalkyl, Aryl, Aryloxy, Arylalkyl. Examples of heterocyclic moieties are: pyrrole, pyrroline, pyrrolidine, pyrazole, pyrazoline, pyrazolidine, imidazole, imidazoline, imidazolidine, triazole, triazoline, triazolidine, tetrazole, furane, dihydrofurane, tetrahydrofurane, furazane (oxadiazole), dioxolane, thiophene, dihydrothiophene, tetrahydrothiophene, oxazole, oxazoline, oxazolidine, isoxazole, isoxazoline, isoxazolidine, thiazole, thiazoline, thiazlolidine, isothiazole, istothiazoline, isothiazolidine, thiadiazole, thiadiazoline, thiadiazolidine, pyridine, piperidine, pyridazine, pyrazine, piperazine, triazine, pyrane, tetrahydropyrane, thiopyrane, tetrahydrothiopyrane, oxazine, thiazine, dioxine, morpholine, purine, pterine, and the corresponding benz-annelated heterocycles, e.g. indole, isoindole, cumarine, cumaronecinoline, isochinoline, cinnoline and the like.

"Hetero atoms" are atoms other than Carbon and Hydrogen, preferably Nitrogen (N), Oxygen (O) or Sulfur (S).

"Halogen" represents Fluoro, Chloro, Bromo or lodo, preferably represents Fluoro, Chloro or Bromo and particularly preferably represents Chloro.

Compounds of formula (I) exist in free or acid addition salt form. In this specification, unless otherwise indicated, language such as "compounds of formula (I)" is to be understood as embracing the compounds in any form, for example free base or acid addition salt form. Salts which are unsuitable for pharmaceutical uses but which can be employed, for example, for the isolation or purification of free compounds of formula (I), such as picrates or perchlorates, are also included. For therapeutic use, only pharmaceutically acceptable salts or free compounds are employed (where applicable in the form of pharmaceutical preparations), and are therefore preferred.

On account of the asymmetrical carbon atom(s) that may be present in the compounds of formula (I) and their salts, the compounds may exist in optically active form or in form of mixtures of optical isomers, e.g. in form of racemic mixtures or diastereomeric mixtures. All optical isomers and their mixtures, including the racemic mixtures, are part of the present invention. Preferred compounds of formula (I) have trans configuration in respect to R⁴ and N

Preferred substituents, preferred ranges of numerical values or preferred ranges of the radicals present in the formula (I) and the corresponding intermediate compounds are defined below.
- n: preferably represents 0, 1 or 2.
- n: particularly preferably represents 1.
- R¹: preferably represents hydrogen or methyl.
- R¹: particularly preferably represents hydrogen.
- R³: preferably represents halogen, C₁₋₄ alkyl.
- R³: particularly preferably represents fluoro or methyl.
- R⁴: preferably represents OH.
- R⁵: preferably represents H.
- R⁶: preferably represents H.
- R²: preferably represents an unsubstituted or substituted heterocycle having 3 - 11 ring atoms and 1 - 4 hetero atoms; the hetero atoms being selected from the group consisting of N, O, S, the substituents being selected from the group consisting of Oxo (=O), Hydroxy, Halogen, Amino, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl, furyl.
- R²: further preferably represents phenyl or substituted phenyl, the substituents being selected from the group consisting of Hydroxy, Amino, Halogen, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl.
- R¹ and R²: together with the nitrogen atom further preferably form unsubstituted or substituted heterocycle having 3 - 11 ring atoms and 0 - 3 additional hetero atoms; the additional hetero atoms being selected from the group consisting of N, O, S; the substituents being selected from the group consisting of Oxo (=O), Hydroxy, Halogen, Amino, Nitro, Cyano, C₁₋₄ Alkyl, C₁₋₄ Alkoxy, C₁₋₄ Alkoxyalkyl, C₁₋₄ Alkoxycarbonyl, C₁₋₄ Alkoxycarbonylalkyl, C₁₋₄ Halogenalkyl, C₆₋₁₀ Aryl, Halogen- C₆₋₁₀ Aryl, C₆₋₁₀ Aryloxy, C₆₋₁₀-Aryl-C₁₋₄ alkyl.
- R²: particularly preferably represents an unsubstituted, a single or twofold substituted heterocycle having 5 - 10 ring atoms and 1 - 3 hetero atoms; the hetero atoms being selected from the group consisting of N, O, S; the substituents being selected from the group consisting of fluoro, chloro, methyl, ethyl, n-, i-propyl, n-, iso-, sec-, tert-butyl, phenyl, tolyl.
- R²: particularly preferably represents an unsubstituted, a single or twofold substituted phenyl, the substituents being selected from the group consisting of fluoro, chloro, bromo.
- R¹ and R²: together with the nitrogen atom further particularly preferably form a single or twofold substituted heterocycle having 5 - 9 ring atoms and 0 - 2 additional hetero atoms; the additional hetero atoms being selected from the group consisting of N, O; the substituents being selected from the group consisting of methyl, ethyl, n-, i-propyl, n-, iso-, sec-, tert-butyl, phenyl, tolyl.

The abovementioned general or preferred radical definitions apply both to the end products of the formula (I) and also, correspondingly, to the starting materials or intermediates required in each case for the preparation. These radical definitions can be combined with one another at will, i.e. including combinations between the given preferred ranges. Further, individual definitions may not apply.

Preference according to the invention is given to compounds of the formula (I) which contain a combination of the meanings mentioned above as being preferred.

Particular preference according to the invention is given to compounds of the formula (I) which contain a combination of the meanings listed above as being particularly preferred.

Very particular preference according to the invention is given to the compounds of the formula (I) which contain a combination of the meanings listed above as being very particularly preferred.

Preferred are compounds of formula (I') 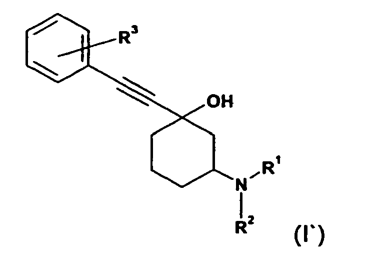 wherein R¹, R², R³ are as defined above.

A further preferred group of compounds of formula (I) are compounds wherein R³ is in the meta-position.

A further preferred group of compounds of formula (I) are compounds wherein the heterocycle of R² is an aromatic heterocycle.

In a further aspect, the invention provides a process for the production of the compounds of formula (I) and their salts, which comprises the step of
a) for the production of a compound of formula (I) wherein i) R⁴ represents hydroxy, R¹ represents hydrogen or C₁-C₄ alkyl and R² represents an unsubstituted or substituted heterocycle or ii) R¹ represents hydrogen or C₁-C₄ alkyl and R² represents aryl or substituted aryl, by reductive amination of a compound of formula (II) 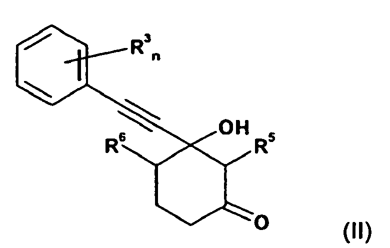 wherein R⁶, R⁵, R³, n are as defined above, with a compound of formula (III) 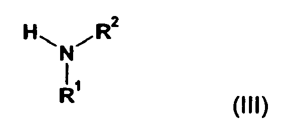 wherein R¹ and R² are as defined above, or
b) for the production of a compound of formula (I) wherein R⁴ represents hydroxy, R¹ and R² together with the nitrogen atom form an unsubstituted or substituted heterocycle, by cyclocondensation of a compound of formula (IV) 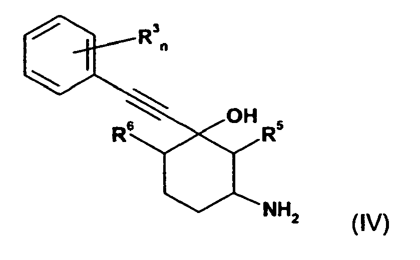
c) for the production of a compound of formula (I) wherein R⁴ represents hydroxy, R¹ and R² together with the nitrogen atom form an unsubstituted or substituted heterocycle, by Michael additon reaction of a compound of formula (V) 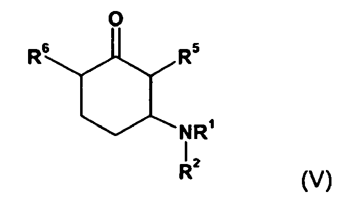 wherein R⁶, R⁵, R², R¹ are as defined above, with a compound of formula (VI) 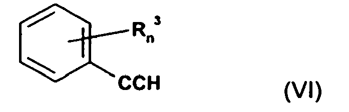 wherein R³ and n are as defined above, or
d) for the production of a compound of formula (I) wherein i) R⁴ and R⁵ form a bond and R⁶ represents hydrogen or C₁-C₄ alkyl or ii) wherein R⁴ and R⁶ form a bond and R⁵ represents hydrogen, by dehydrating a compound of formula (I) wherein R⁴ is hydroxyl R⁵ and R⁶ are hydrogen or C₁-C₄ alkyl,
and recovering the resulting compound of formula (I) in free base or acid addition salt form.

The reaction of processes a) b), c) and d) can be effected according to conventional methods, e.g. as described in the examples. Process c) is preferred for compounds wherein R¹ and R² together with the nitrogen form a heterocycle, especially preferably a substituted heterocycle. Process d) might be a side reaction of a previous reaction step, depending on pH, temperature and nature of substituents. In this case the compounds of formula one are isolated according to conventional methods, e.g. chromatography.

The reaction of process d) generally leads to a mixture of a compound of formula (I) wherein R⁴ forms a single bond with R⁵ and a compound of formula I wherein R⁴ forms a single bond with R⁶, which are subsequent separated according to conventional methods. , e.g. as described in WO 03/047581.

A so obtained compound of formula (I) can be converted into another compound of formula (I) according to conventional methods.

Generally, the starting materials for manufacturing compounds of formula (I) are known or obtainable according to known processes. Certain starting materials, which are useful for the production of compounds of formula (I), are novel.

A compound of formula (II) 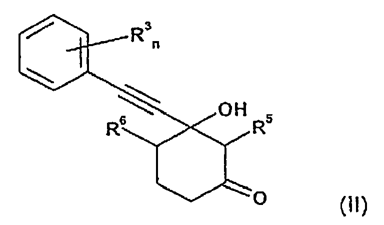 wherein R⁶, R⁵, R³, n are as defined above for compounds of formula (I).

A compound of formula (V) 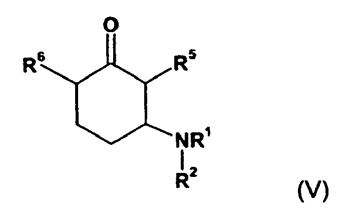 wherein R⁶, R⁵, R², R¹ are as defined above, with a compound of formula (VI)

A compound of formula (V) is obtainable by reacting a cycloehexenone of formula (VII) 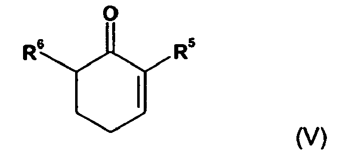 wherein R⁶, R⁵, are as defined above, with an amine of formula (III) under basic conditions.

The following considerations apply to the individual reaction steps described above:
a) One or more functional groups, for example carboxy, hydroxy, amino, or mercapto, may need to be protected in the starting materials by protecting groups. The protecting groups employed may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter. The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene, "Protective Groups in Organic Synthesis", Wiley, New York 1981, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/I, Georg Thieme Verlag, Stuttgart 1974, in H.-D. Jakubke and H. Jescheit, "Aminosäuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.
b) Acid addition salts may be produced from the free bases in known manner, and vice-versa. Compounds of formula (I) in optically pure form can be obtained from the corresponding racemates according to well-known procedures, e.g. HPLC with chiral matrix. Alternatively, optically pure starting materials can be used.
c) Stereoisomeric mixtures, e.g. mixtures of diastereomers, can be separated into their corresponding isomers in a manner known per se by means of suitable separation methods. Diastereomeric mixtures for example may be separated into their individual diastereomers by means of fractionated crystallization, chromatography, solvent distribution, and similar procedures. This separation may take place either at the level of a starting compound or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.
d) Suitable diluents for carrying out the above- described are especially inert organic solvents. These include, in particular, aliphatic, alicyctic or aromatic, optionally halogenated hydrocarbons, such as, for example, benzine, benzene, toluene, xylene, chlorobenzene, dichlorobenzene, petroleum ether, hexane, cyclohexane, dichloromethane, chloroform, carbon tetrachloride; ethers, such as diethyl ether, diisopropyl ether, dioxane, tetrahydrofuran or ethylene glycol dimethyl ether or ethylene glycol diethyl ether; ketones, such as acetone, butanone or methyl isobutyl ketone; nitriles, such as acetonitrile propionitrile or butyronitrile; amides, such as N,N-dimethylformamide, N,N-dimethylacetamide, N-methyl-formanilide, N-methyl-pyrrolidone or hexamethylphosphoric triamide; esters, such as methyl acetate or ethyl acetate, sulphoxides, such as dimethyl sulphoxide, alcohols, such as methanol, ethanol, n- or i-propanol, ethylene glycol monomethyl ether, ethylene glycol monoethyl ether, diethyelene glycol monomethyl ether, diethylene glycol monoethyl ether. Further, mixtures of diluents may be employed. Depending on the starting materials, reaction conditions and auxiliaries, water or diluents constaining water may be suitable. It is also possible to use one a starting material as diluent simultaneously.
e) Reaction temperatures can be varied within a relatively wide range. In general, the processes are carried out at temperatures between 0°C and 150°C, preferably between 10°C and 120°C. Deprotonation reactions can be varied within a relatively wide range. In general, the processes are carried out at temperatures between -150°C and +50°C, preferably between - 75°C and 0°C.
f) The reactions are generally carried out under atmospheric pressure. However, it is also possible to carry out the processes according to the invention under elevated or reduced pressure - in general between 0.1 bar and 10 bar.
g) Starting materials are generally employed in approximately equimolar amounts. However, it is also possible to use a relatively large excess of one of the components. The reaction is generally carried out in a suitable diluent in the presence of a reaction auxiliary, and the reaction mixture is generally stirred at the required temperature for a number of hours.
h) Work-up is carried out by customary methods (cf. the Preparation Examples).

Compounds of formula (I) and their pharmaceutically acceptable acid addition salts, hereinafter referred to as agents of the invention, exhibit valuable pharmacological properties and are therefore useful as pharmaceuticals.

In particular, the agents of the invention exhibit a marked and selective modulating, especially antagonistic, action at human metabotropic glutamate receptors (mGluRs). This can be determined in vitro for example at recombinant human metabotropic glutamate receptors, especially PLC-coupled subtypes thereof such as mGluR5, using different procedures like, for example, measurement of the inhibition of the agonist induced elevation of intracellular Ca²⁺ concentration in accordance with L. P. Daggett et al., Neuropharm. Vol. 34, pages 871-886 (1995), P. J. Flor et al., J. Neurochem. Vol. 67, pages 58-63 (1996) or by determination to what extent the agonist induced elevation of the inositol phosphate turnover is inhibited as described by T. Knoepfel et al., Eur. J. Pharmacol. Vol. 288, pages 389-392 (1994), L. P. Daggett et al., Neuropharm. Vol. 67, pages 58-63 (1996) and references cited therein. Isolation and expression of human mGluR subtypes are described in US-Patent No. 5,521,297. Selected agents of the invention show IC50 values for the inhibition of the agonist (e.g. glutamate or quisqualate) induced elevation of intracellular Ca2+ concentration or the agonist (e.g. glutamate or quisqualate) induced inositol phosphate turnover, measured in recombinant cells expressing hmGluR5a of about 1nM to about 50 µM.

The agents of the invention are therefore useful in the prevention, treatment or delay of progression of disorders associated with irregularities of the glutamatergic signal transmission, of the gastro-intestinal and urinary tract and of nervous system disorders mediated full or in part by mGluR5.

Disorders associated with irregularities of the glutamatergic signal transmission are for example epilepsy, cerebral ischemias, especially acute ischemias, ischemic diseases of the eye, muscle spasms such as local or general spasticity, skin disorders, obesity disorders and, in particular, convulsions or pain.

Disorders of the gastro-intestinal tract include post-operative ileus, functional gastro-intestinal disorders (FGID) as for example functional dyspepsia (FD), gastro-esophageal reflux disease (GERD), irritable bowel syndrome (IBS), functional bloating, functional diarrhea, chronic constipation, functional disturbancies of the biliary tract as well as other conditions according to Gut 1999; Vol. 45 Suppl. II.

Disorders of the Urinary Tract comprise conditions associated with pain and/or discomfort of the urinary tract and overactive bladder (OAB).

Nervous system disorders mediated full or in part by mGluR5 are for example acute, traumatic and chronic degenerative processes of the nervous system, such as Parkinson's disease, senile dementia, Alzheimer's disease, Huntington's chorea, amyotrophic lateral sclerosis, multiple sclerosis and fragile X syndrome, psychiatric diseases such as schizophrenia and anxiety, depression, pain, itch and drug abuse. Anxiety related disorders includes panic disorders, social anxiety, obsessive compulsive disorders (OCD), post traumatic stress disorders (ATSD), generalized anxiety disorders (GAD), phobias.

The usefulness of the agents of the invention in the prevention, treatment or delay of progression of the above-mentioned disorders can be confirmed in a range of standard tests including those indicated below:
Activity of the agents of the invention in anxiety can be demonstrated in standard models such as the stress-induced hyperthermia in mice [cf. A. Lecci et al., Psychopharmacol. 101, 255-261]. At doses of about 0.1 to about 30 mg/kg p.o., selected agents of the invention reverse the stress-induced hyperthermia.

At doses of about 4 to about 50 mg/kg p.o., selected agents of the invention show reversal of Freund complete adjuvant (FCA) induced hyperalgesia [cf. J. Donnerer et al., Neuroscience 49, 693-698 (1992) and C.J. Woolf, Neuroscience 62, 327-331 (1994)].

For all the above mentioned indications, the appropriate dosage will of course vary depending upon, for example, the compound employed, the host, the mode of administration and the nature and severity of the condition being treated. However, in general, satisfactory results in animals are indicated to be obtained at a daily dosage of from about 0.5 to about 100 mg/kg animal body weight. In larger mammals, for example humans, an indicated daily dosage is in the range from about 5 to 1500 mg, preferably about 10 to about 1000 mg of the compound conveniently administered in divided doses up to 4 times a day or in sustained release form.

In accordance with the foregoing, the present invention also provides an agent of the invention for use as a pharmaceutical, e.g. in the prevention, treatment or delay of progression of disorders associated with irregularities of the glutamatergic signal transmission, of the gastro-intestinal and urinary tract and of nervous system disorders mediated full or in part by mGluR5.

The invention also provides the use of an agent of the invention, in the prevention, treatment or delay of progression of disorders associated with irregularities of the glutamatergic signal transmission, of the gastro-intestinal and urinary tract and of nervous system disorders mediated full or in part by mGluR5.

Furthermore the invention provides the use of an agent of the invention for the manufacture of a pharmaceutical composition designed for the prevention, treatment or delay of progression of disorders associated with irregularities of the glutamatergic signal transmission, of the gastro-intestinal and urinary tract and of nervous system disorders mediated full or in part by mGluR5.

In a further aspect the invention relates to a method of treating disorders mediated full or in part by mGluR5, which method comprises administering to a warm-blooded organism in need of such treatment a therapeutically effective amount of an agent of the invention.

Moreover the invention relates to a pharmaceutical composition comprising an agent of the invention in association with one or more pharmaceutical carrier or one or more pharmaceutically acceptable diluent.

The pharmaceutical compositions according to the invention are compositions for enteral, such as nasal, rectal or oral, or parenteral, such as intramuscular or intravenous, administration to warm-blooded animals (human beings and animals) that comprise an effective dose of the pharmacological active ingredient alone or together with a significant amount of a pharmaceutically acceptable carrier. The dose of the active ingredient depends on the species of warm-blooded animal, body weight, age and individual condition, individual pharmacokinetic data, the disease to be treated and the mode of administration.

The pharmaceutical compositions comprise from approximately 1% to approximately 95%, preferably from approximately 20% to approximately 90%, active ingredient. Pharmaceutical compositions according to the invention may be, for example, in unit dose form, such as in the form of ampoules, vials, suppositories, dragées, tablets or capsules.

The pharmaceutical compositions of the present invention are prepared in a manner known per se, for example by means of conventional dissolving, lyophilizing, mixing, granulating or confectioning processes.

The preferred agents of the invention include the (±)-(1R,3R)-3-(4-Chloro-phenylamino)-1-(3-chloro-phenylethynyl)-cyclohexanol free base or pharmaceutically acceptable acid addition salt form.

(±)-(1R,3R)-3-(4-Chloro-phenylamino)-1-(3-chloro-phenylethynyl)-cyclohexanol inhibits the quisqualate-induced inositol phosphate turnover in hmGluR5 expressing cells with an IC₅₀ concentration of 1600 nM.

With (±)-(1R,3R)-3-(4-Chloro-phenylamino)-(3-chloro-phenylethynyl)-cyclohexanol, a stress-induced hyperthermia of 0.98 +/- 0.08 °C was reduced to 0.66 +/- 0.06 °C at 1 mg/kg p.o., to 0.43 +/- 10 °C at 3 mg/kg p.o.; to 0.58 +/- 0.06 °C at 10 mg/kg p.o. and to 0.33 +/- 0.04 °C at 30 mg/kg p.o. (p < 0.05; p < 0.0011; p < 0.01; p < 0.001 respectively).

Further, properly isotope-labeled agents of the invention exhibit valuable properties as histopathological labeling agents, imaging agents and/or biomarkers, hereinafter "markers", for the selective labeling of the metabotropic glutamate receptor subtype 5 (mGlu5 receptor). More particularly the agents of the invention are useful as markers for labeling the central and peripheral mGlu5 receptors *in vitro* or *in vivo.* In particular, compounds of the invention which are properly isotopically labeled are useful as PET markers. Such PET markers are labeled with one or more atoms selected from the group consisting of ¹¹C, ¹³N, ¹⁵O, ¹⁸F.

The agents of the invention are therefore useful, for instance, for determining the levels of receptor occupancy of a drug acting at the mGlu5 receptor, or diagnostic purposes for diseases resulting from an imbalance or dysfunction of mGlu5 receptors, and for monitoring the effectiveness of pharmacotherapies of such diseases.

In accordance with the above, the present invention provides an agent of the invention for use as a marker for neuroimaging.

In a further aspect, the present invention provides a composition for labeling brain and peripheral nervous system structures involving mGlu5 receptors *in vivo* and *in vitro* comprising an agent of the invention.

In still a further aspect, the present invention provides a method for labeling brain and peripheral nervous system structures involving mGlu5 receptors *in vitro* or *in vivo,* which comprises contacting brain tissue with an agent of the invention.

The method of the invention may comprise a further step aimed at determining whether the agent of the invention labeled the target structure. Said further step may be effected by observing the target structure using positron emission tomography (PET) or single photon emission computed tomography (SPECT), or any device allowing detection of radioactive radiations.

The following non-limiting Examples illustrate the invention. A list of Abbreviations used is given below.
- BOC: tert-butoxycarbonyl
- n-BuLi: *n*-butyl lithium
- DCM: dichloromethane
- DMF: N,N'-dimethylformamide
- EDC: 1-ethyl-3-[3-(dimethylamino)propyl]-carbodiimide hydrochloride
- EtOAc: ethylacetate
- h: hours
- HCl: hydrochloric acid
- HOBt: hydroxybenzotriazole
- HPLC: high pressure liquid chromatography
- min: minutes
- Mp: melting point
- MS: mass spectroscopy
- MTBE: methyl-tert.-butylether
- Rf: retention factor (Thin Layer Chromatography)
- Rt: retention time
- rt: room temperature
- TFA: trifluoroacetic acid
- THF: tetrahydrofuran

### Example 1: (±)-(1R,3R)-3-(4-Chloro-phenylamino)-1-(3-chloro-phenylethynyl)-cyclohexanol

A solution of (±)-3-(3-Chloro-phenylethynyl)-3-hydroxy-cyclohexanone (500 mg, 2 mmol), 4-chloroaniline (256 mg, 2 mmol) and acetic acid (121 mg, 2 mmol) in DCM (30 ml) is treated with sodiumtriacetoxy borohydride (597 mg, 2.8 mmol) and stirred for 3 h at room temperature. The mixture is diluted with EtOAc, washed with sodium bicarbonate and brine, dried (Na2SO4) and the solvent evaporated to afford 324 mg of a red oil. This crude product was purified by chromatography on silica gel and treated with an excess of HCl in diethyl ether which afforded after evaporation of the solvent the hydrochloride of the title compound as an amorphous orange powder (112 mg, 14%). Mp: 153-163°C. MS (LC/MS): 361.3 [M+H].

The starting material was prepared as described hereafter:
i) (±)-7-(3-Chloro-phenylethynyl)-1,4-dioxa-spiro[4.5]decan-7-ol: 1-Chloro-3-ethynyl-benzene (6.4 g, 47.2 mmol) was dissolved in THF (250 ml) and cooled to -20°C. A solution of n-BuLi in hexanes (29.5 ml, 1.6 M, 47.2 mmol) was added within 1 h and the solution stirred for an additional hour at this temperature. The mixture was then cooled to -78° and a solution of 1,4-Dioxa-spiro[4.5]decan-7-one (4.9 g, 31.4 mmol) in THF (100 ml) was added dropwise within 30'. The cooling bath was removed and the mixture was allowed to reach room temperature. EtOAc was added, the mixture was washed with aqueous sodium bicarbonate and brine, dried and evaporated to afford an orange oil (8.43 g). Chromatography on silica gel afforded the title compound as a yellow oil (4.65 g, 50%).
ii) (±)-3-(3-Chloro-phenylethynyl)-3-hydroxy-cyclohexanone: A solution of (±)-7-(3-Chloro-phenylethynyl)-1,4-dioxa-spiro[4.5]decan-7-ol (4.6 g, 15.88 mmol) and p-TsOH (61 mg) in acetone (100 ml) was stirred at room temperature for 24 h. Dilution with EtOAc, washing with aqueous sodium bicarbonate and brine, drying and evaporation of the solvents afforded the crude product as a yellow oil (4.53 g). Chromatography on silica gel led to the pure title compound as a slightly yellowish oil (3.60 g, 91%).

Following the same procedure, the following compounds can be obtained:

### Example 1.1: ((±)-1R,3R)-1-(3-Chloro-phenylethynyl)-3-(3,4-difluoro-phenylamino)-cyclohexanol

MS (LC/MS): 362.3 [M+H]
TLC Rf: 0.11 (EtOAc/cyclohexane 1: 1)

### Example 1.2: ((±)-1R,3R)-1-(3-Chloro-phenylethynyl)-3-(4-phenyl-thiazol-2-ylamino)-cyclohexanol

MS (LC/MS): 410.4 [M+H]
TLC Rf: 0.60 (EtOAc/cyclohexane 1:1)

### Example 1.3: ((±)-1R,3R)-3-(4-Chloro-phenylamino)-1-m-tolylethynyl-cyclohexanol

MS (LC/MS): 340.5 [M+H]
TLC Rf: 0.60 (EtOAc/cyclohexane 1:1)

### Example 1.4: ((±)-1R,3R)-3-(3,4-Difluoro-phenylamino)-1-m-tolylethynyl-cyclohexanol

MS (LC/MS): 342.1 [M+H]
TLC Rf: 0.59 (EtOAc/cyclohexane 1:1)

### Example 1.5: ((±)-1R,3R)-3-(5-Methyl-1H-pyrazol-3-ylamino)-1-m-tolylethynyl-yclohexanol

MS (LC/MS): 310.1 [M+H]
TLC Rf: 0.41 (EtOAc/MeOH/Et₃N 90:9:1)

### Example 1.6: ((±)-1R,3R)-1-(3-Chloro-phenylethynyl)-3-(pyridin-2-ylamino)-cyclohexanol

MS (LC/MS): 327.4 [M+H]
TLC Rf: 0.35 (EtOAc)

### Example 1.7: ((±)-1R,3R)-1-(3-Chloro-phenylethynyl)-3-(pyridin-3-ylamino)-cyclohexanol

MS (LC/MS): 327.4 [M+H]
TLC Rf: 0.34 (EtOAc)

### Example 1.8: ((±)-1R,3R) -1-(3-Chloro-phenylethynyl)-3-(quinoxalin-6-ylamino)-cyclohexanol

MS (LC/MS): 378.3 [M+H]
TLC Rf: 0.37 (EtOAc/hexane 1:1)

### Example 1.9: ((±)-1R,3R)-1-(3-Chloro-phenylethynyl)-3-(2,3-dihydro-benzo[1,4]dioxin-6-ylamino)-cyclohexanol

MS (LC/MS): 384.3 [M+H]
TLC Rf: 0.46 (EtOAc/hexane 2:3)

### Example 1.10: ((±)-1R,3R)-1-(3-Chloro-phenylethynyl)-3-(5-methyl-1H-pyrazol-3-ylamino)-cyclohexanol

MS (LC/MS): 330.4 [M+H]
TLC Rf: 0.54 (EtOAc/hexane 3:2)

### Example 1.11: ((±)-1R,3R)-1-(3-Chloro-phenylethynyl)-3-(5-phenyl-1H-pyrazol-3-ylamino)-cyclohexanol

MS (LC/MS): 392.4 [M+H]
TLC Rf: 0.33 (EtOAc/hexane 3:2)

### Example 1.12: ((±)-1R,3R)-1-(3-Chloro-phenylethynyl)-3-(2,2-difluoro-benzo[1,3]dioxol-5-ylamino)-cyclohexanol

MS (LC/MS): 406.1 [M+H]
TLC Rf: 0.41 (EtOAc/cyclohexane 1:9)

### Example 1.13: ((±)-1R,3R)-1-(3-Chloro-phenylethynyl)-3-(quinolin-8-ylamino)-cyclohexanol

MS (LC/MS): 377.1 [M+H]
TLC Rf: 0.45 (EtOAc/hexane 1:2)

### Example 1.14: ((±)-1R,3R)-1-(3-Chloro-phenylethynyl)-3-(2,3-dihydro-imidazo[1,2-b]pyrazol-1-yl)-cyclohexanol

MS (LC/MS): 342.1 [M+H]
TLC Rf: 0.32 (EtOAc/hexane 2:1)

### Example 1.15: (±)-(1S,3S)-(1R,3R)-1-(3-Chloro-phenylethynyl)-3-(1H-indazol-3-ylamino)-cyclohexanol

MS (LC/MS): 366.3 [M+H]
TLC Rf: 0.31 (EtOAc/cyclohexane 1:1)

### Example 1.16: [3-(3-Chloro-phenylethynyl)-cyclohexyl]-(5-methyl-4H-pyrazol-3-yl)-amine

MS (LC/MS): 312.3 [M+H]
TLC Rf: 0.53 (EtOAc/hexane 2:3)

### Example 1.17: (±)-(1S,3S)-(1R,3R)-3-(Benzo[1,2,5]thiadiazol-5-ylamino)-1-(3-chloro-phenylethynyl)-cyclohexanol

MS (LC/MS): 384.1 [M+H]
TLC Rf: 0.51 (EtOAc/hexane 1:4)

### Example 1.18: (±)-(1S,3S)-(1R,3R)-1-(3-Chloro-phenylethynyl)-3-(2-methyl-5-phenyl-2H-pyrazol-3-ylamino)-cyclohexanol

MS (LC/MS): 406.1 [M+H]
TLC Rf: 0.51 (EtOAc/cyclohexane 1:1)

### Example 1.19: (±)-(1S,3S)-(1R,3R)-1-(3-Chloro-phenylethynyl)-3-(5-furan-2-yl-1H-pyrazol-3-ylamino)-cyclohexanol

MS (LC/MS): 382.1 [M+H]
TLC Rf: 0.15 (EtOAc/cyclohexane 1:1)

### Example 1.20: (±)-(1S,3S)-(1R,3R)-1-(3-Chloro-phenylethynyl)-3-(2,2,3,3-tetrafluoro-2,3-dihydro-benzo[1,4]dioxin-6-ylamino)-cyclohexanol

MS (LC/MS): 456.1 [M+H]
TLC Rf: 0.33 (EtOAc/cyclohexane 1:9)

### Example 1.21: (±)-(1S,3S)-1-(3-Chloro-phenylethynyl)-3-(6-methoxy-pyridin-2-ylamino)-cyclohexanol

MS (LC/MS): 357.1 [M+H]
TLC Rf: 0.40 (EtOAc/cyctohexane 1:3)

### Example 1.22: (±)-(1S,3S)-1-(3-Chloro-phenylethynyl)-3-(2,3-dihydro-[1,4]dioxino[2,3-b]pyridin-6-ylamino)-cyclohexanol

MS (LC/MS): 385.1 [M+H]

### Example 1.23: (±)-(1S,3S)-1-(3-Chloro-phenylethynyl)-3-(5-fluoro-pyridin-2-ylamino)-cyclohexanol

MS (LC/MS): 345.1 [M+H]
TLC Rf: 0.29 (EtOAc/cyclohexane 1:4)

### Example 1.24: (1S,3S)-1-(3-Chloro-phenylethynyl)-3-(2,2-difluoro-benzo[1,3]dioxol-5-ylamino)-cyclohexanol

[α]_{D} = -154.5° (c=1.1, MeOH)
MS (LC/MS): 406.1 [M+H]

### Example 1.25: (1R,3R)-1-(3-Chloro-phenylethynyl)-3-(2,2-difluoro-benzo[1,3]dioxol-5-ylamino)-cyclohexanol

[α]_{D} = 158.4° (c=1, MeOH)
MS (LC/MS): 382.1 [M+H]

### Example 1.26: (1S,3S)-1-(3-Chloro-phenylethynyl)-3-(5-furan-2-yl-1H-pyrazol-3-ylamino)-cyclohexanol

[α]_{D} = -189° (c=0.5, MeOH)
M.p. = 83-88°C

### Example 1.27: (1R,3R)-1-(3-Chloro-phenylethynyl)-3-(5-furan-2-yl-1H-pyrazol-3-ylamino)-cyclohexanol

[α]_{D} = 186.4° (c=0.5, MeOH)
M.p. = 78-85°

### Example 1.28: (1S,3S)-1-(3-Chloro-phenylethynyl)-3-(2,2,3,3-tetrafluoro-2,3-dihydro-benzo[1,4]dioxin-6-ylamino)-cyclohexanol

[α]_{D} = -156.8° (c=0.37, MeOH)
TLC Rf: 0.33 (EtOAc/cyclohexane 1:9)

### Example 1.29: (1R,3R)-1-(3-Chloro-phenylethynyl)-3-(2,2,3,3-tetrafluoro-2,3-dihydro-benzo[1,4]dioxin-6-ylamino)-cyclohexanol

[α]_{D} = 133.8° (c=0.53, MeOH)
TLC Rf: 0.33 (EtOAclcyclohexane 1:9)

### Example 1.30: (1R,3R)-1-(3-Chloro-phenylethynyl)-3-(quinoxalin-6-ylamino)-cyclohexanol.

A solution of [(1R,3R)-3-(3-Chloro-phenylethynyl)-3-(4-methoxy-benzyloxyycyclohexyl]-quinoxalin-6-yl-amine (24.9 mg) in MeOH (1 ml) was cooled to 0° and treated with 4M HCl in dioxane (1.00 ml) in a dropwise manner. The solution was stirred at room temperature for 3 h, then poured onto ice/aqueous conc NH₄OH and the mixture extracted with Et₂O. The organic phase was dried and evaporated under reduced pressure. Purification by preparative thin layer chromatography afforded 10 mg of the title compound (53%). [α]_{D} = 485° (c=0.5, MeOH). MS (LC/MS): 378 [M+H].

The starting material was prepared as described hereafter:
i) (3-Oxo-cyclohexyl)-carbamic acid tert-butyl ester: A solution of 2-cyclohexen-1-on (14 ml, 150 mmol) and t-butylcarbamate (17 g, 145.11 mmol) in DCM (30 ml) was treated with bismuth nitrate pentahydrate (14 g, 28.8 mmol) and stirred at room temperature for 21 h. Dilution with further DCM, filtration over hyflo, washing of the filtrate with sodium bicarbonate solution and brine, drying of the organic phase with Na2SO4, filtration and evaporation of the solvent afforded 22.1 g of the crude product. Chromatography on silica gel (EtOAc/cyclohexanol 3:7), followed by crystallization from the same solvent system afforded (3-oxo-cyclohexyl)-carbamic acid tert-butyl ester (14.43 g, 47%).
ii) rac-[(1R,3R)-3-(3-Chloro-phenylethynyl)-3-hydroxy-cyclohexyl]-carbamic acid tert-butyl ester: 1-Chloro-3-ethynyl-benzene (9.0 ml, 71 mmol) was dissolved in THF (250 ml) and cooled to - 20°. A solution of n-BuLi in hexanes (44 ml, 1.6 M, 70 mmol) was added dropwise and the mixture stirred at -20° for 2 h. After cooling to -60°, a solution of (3-oxo-cyclohexyl)-carbamic acid tert-butyl ester (15.15 g, 71 mmol) in THF (100 ml) was added slowly. The mixture was allowed to reach room temperature and then stirred for 16 h. Dilution of the mixture with EtOAc, washing with sodium bicarbonate solution and brine, drying of the organic phase with Na2SO4, filtration and evaporation of the solvent afforded a crude product as a mixture of cis and trans isomers. Careful chromatography on silica gel with EtOAc/cyclohexane 4:6 afforded first the desired rac-(R,R) isomer ('trans' for -OH and -NH, 2.48 g, 10%), followed by the rac-(R,S) isomer ('cis' for -OH and -NH, 8 g).
iii) (+)-[(1R,3R)-3-(3-Chloro-phenylethynyl)-3-hydroxy-cyclohexyl]-carbamic acid tert-butyl ester: rac-[(1R,3SR-3-(3-Chloro-phenylethynyl)-3-hydroxy-cyclohexyl]-carbamic acid tert-butyl ester (2.26 g) was separated into its enantiomers via HPLC using Chiralcel OD as stationary phase and hexanes/EtOH as eluent. 1.1 g of each enantiomer was isolated. [α]_{D} = +98.5° (c=0.5, MeOH) and -94.3° (c=0.6, MeOH), respectively.
iv) [(1R,3R)-3-(3-Chloro-phenylethynyl)-3-(4-methoxy-benzyloxy)-cyclohexyl]-carbamic acid tert-butyl ester: (+)-[(1R,3R)-3-(3-Chloro-phenylethynyl)-3-hydroxy-cyclohexyl]-carbamic acid tert-butyl ester (3.50 g) was dissolved in THF (35 ml) and treated with NaH (800 mg) for 40 min. Nal (15.4 mg) and 4-methoxybenzyl bromide (2.51 g) were added and the mixture stirred for 16 h at room temperature. All volatiles were evaporate under reduced pressure, the residue triturated with silicagel (30 g) and cyclohexanol, and then filtered. The product was eluted from the silicagel with cyclohexanol/EtOAc 1:1 to affort 4.7 g (70%) of the desired 4-methoxybenzyl ether.
v) (1R,3R)-3-(3-Chloro-phenylethynyl)-3-(4-methoxy-benzyloxy)-cyclohexylamine: [(1R,3R)-3-(3-Chloro-phenylethynyl)-3-(4-methoxy-benzyloxy)-cyclohexyl]-carbamic acid tert-butyl ester (1.6 g) was dissolved in THF (27 ml) and treated with a solution of p-toluenesulfonic acid (660 mg) in EtOH (5 ml) for 9 h at reflux temperature. The mixture was distributed between cold 1 M Na₂CO₃ and EtOAc, the phases separated, the organic phase dried over Na₂SO₄ and evaporated. Flash chromatography afforded the desired primary amine (0.58 g, 46%).
vi) [(1R,3R)-3-(3-Chloro-phenylethynyl)-3-(4-methoxy-benzyloxy)-cyclohexyl]-quinoxalin-6-yl-amine: A solution of (1 R,3R)-3-(3-Chloro-phenylethynyl)-3-(4-methoxy-benzyloxy)-cyclohexylamine (34 mg), 6-bromoquinoxaline (23 mg), NaOt-Bu (13 mg), Pd₂(dba)₃ CHCl₃ (1.9 mg) and BINAP (3.5 mg) in de-gassed toluene (2 ml) was stirred under Ar atmosphere for 1.5 h at 100°. The mixture was distributed between cold 1 M Na₂CO₃ and EtOAc, the phases separated, the aqueous phase ectracted with EtOAc, the combined organic phases dried over Na₂SO₄ and evaporated. Chromatography afforded 38 mf of the desired product (83%).

Following the same procedure, the following compounds can be obtained:

### Example 1.31: (1S,3S)-1-(3-Chloro-phenylethynyl)-3-(quinoxalin-6-ylamino)-cyclohexanol

MS (LC/MS): 378 [M+H]
TLC Rf: 0.14 (EtOAc/cyclohexane 1:1)

### Example 1.32: (1S,3S)-1-(3-Chloro-phenylethynyl)-3-(pyridin-2-ylamino)-cyclohexanol

[α]_{D} = 193.8° (c=0.55, MeOH)
MS (LC/MS): 327 [M+H]

### Example 1.33: (1R,3R)1-(3-Chloro-phenylethynyl)-3-(pyridin-2-ylamino)-cyclohexanol

[α]_{D} = 179.8° (c=0.5, MeOH)
MS (LC/MS): 327 [M+H]

### Example 1.34: (1R,3R)-1-(3-Chloro-phenylethynyl)-3-(quinolin-8-ylamino)-cyclohexanol

[α]_{D} = 6.0° (c=0.5, MeOH)
MS (LC/MS): 377 [M+H]

### Example 1.35: (1R,3R)-1-(3-Chloro-phenylethynyl)-3-(2,3-dihydro-[1,4]dioxino[2,3-b]pyridin-6-ylamino)-cyclohexanol

[α]_{D} = 242° (c=0.23, MeOH)
MS (LC/MS): 385 [M+H]

### Example 1.36: (1R,3R)-1-(3-Chloro-phenylethynyl)-3-(5-fluoro-pyridin-2-ylamino)-cyclohexanol

MS (LC/MS): 345 [M+H]
TLC Rf: 0.34 (EtOAc/cyclohexane 1:2)

### Example 1.37: (1R,3R)-1-(3-Chloro-phenylethynyl)-3-(pyrimidin-2-ylamino)-cyclohexanol

MS (LC/MS): 328 [M+H]
TLC Rf: 0.21 (EtOAc/cyclohexane 1:1)

### Example 1.38: (1R,3R)-1-(3-Chloro-phenylethynyl)-3-(5-trifluoromethyl-pyridin-2-ylamino)-cyclohexanol

MS (LC/MS): 395 [M+H]
TLC Rf: 0.45 (EtOAc/cyclohexane 1:2)

### Example 1.39: (1R,3R)-1-(3-Chloro-phenylethynyl)-3-(pyrazin-2-ylamino)-cyclohexanol

MS (LC/MS): 328 [M+H]
TLC Rf: 0.17 (EtOAc/cyclohexane 1:1)

### Example 1.40: (1R,3R)-1-(3-Chloro-phenylethynyl)-3-(6-methoxy-pyridin-2-ylamino)-cyclohexanol

MS (LC/MS): 357 [M+H]
TLC Rf: 0.28 (EtOAc/cyclohexane 1:4)

### Example 1.41: (1R,3R)-1-(3-Chloro-phenylethynyl)-3-(3-chloro-pyridin-2-ylamino)-cyclohexanol

MS (LC/MS): 361 [M+H]
TLC Rf: 0.11 (EtOAc/cyclohexane 1:4)

### Example 1.42: (1R,3R)-1-(3-Chloro-phenylethynyl)-3-(5-chloro-pyridin-2-ylamino)-cyclohexanol

MS (LC/MS): 361 [M+H]
TLC Rf: 0.12 (EtOAc/cyclohexane 1:4)

### Example 1.43: (1R,3R)-1-(3-Chloro-phenylethynyl)-3-(4-chloro-pyridin-2-ylamino)-cyclohexanol

MS (LC/MS): 361 [M+H]
TLC Rf: 0.10 (EtOAc/cyclohexane 1:4)

### Example 1.44: (1R,3R)-1-(3-Chloro-phenylethynyl)-3-(6-chloro-pyridin-2-ylamino)-cyclohexanol

MS (LC/MS): 361 [M+H]
TLC Rf: 0.14 (EtOAc/cyclohexane 1:4)

### Example 1.45: (1R,3R)-1-(3-Chloro-phenylethynyl)-3-(pyridazin-3-ylamino)-cyclohexanol

MS (LC/MS): 328 [M+H]
TLC Rf: 0.14 (EtOAc)

### Example 1.46: (1R,3R)-1-(3-Chloro-phenylethynyl)-3-(5-methyl-pyridin-2-ylamino)-cyclohexanol

MS (LC/MS): 341 [M+H]
TLC Rf: 0.11 (EtOAc/cyclohexane 1:2)

### Example 1.47: (1R,3R)-1-(3-Chloro-phenylethynyl)-3-(pyrimidin-4-ylamino)-cyclohexanol

MS (LC/MS): 328 [M+H]
TLC Rf: 0.15 (EtOAc/cyclohexane 1:2)

### Example 1.48: (1R,3R)-1-(3-Chloro-phenylethynyl)-3-(6-chloro-pyridin-3-ylamino)-cyclohexanol

MS (LC/MS): 362 [M+H]
TLC Rf: 0.22 (EtOAc/hexane 1:3)

### Example 1.49: :(1R,3R)-1-(3-Chloro-phenylethynyl)-3-(5-chloro-pyridin-3-ylamino)-cyclohexanol

MS (LC/MS): 362 [M+H]
TLC Rf: 0.31 (EtOAc/hexane 1:3)

### Example 1.50: (1R,3R)-1-(3-Chloro-phenylethynyl)-3-(pyrimidin-5-ylamino)-cyclohexanol

MS (LC/MS): 328 [M+H]
TLC Rf: 0.18 (EtOAc/hexane 2:1)

### Example 1.51: (1R,3R)-1-(3-Chloro-phenylethynyl)-3-(6-trifluoromethyl-pyridin-2-ylamino)-cyclohexanol

MS (LC/MS): 395 [M+H]
TLC Rf: 0.19 (EtOAc/cyclohexane 1:4)

### Example 1.52: (1R,3R)-1-(3-Chloro-phenylethynyl)-3-(pyrimidin-5-ylamino)-cyclohexanol

MS (LC/MS): 328 [M+H]
TLC Rf: 0.24 (EtOAc)

### Example 1.53: (1R,3R)-1-(3-Chloro-phenylethynyl)-3-(5-fluoro-pyrimidin-2-ylamino)-cyclohexanol

MS (LC/MS): 346 [M+H]
TLC Rf: 0.61 (EtOAc/cyclohexane 1:1)

Example 2: (1R,3R)-1-(3-Chloro-phenylethynyl)-3-imidazol-1-yl-cyclohexanol: The pH of a solution of (1R,3R)-3-amino-1-(3-chloro-phenylethynyl)-cyclohexanol (268 mg, 1.07 mmol) in water (0.5 ml) is adjusted to pH=2 by addition of an aqueous 85% solution of H3PO4. Paraformaldehyde (32 mg, 1.07 mmol), Glyoxal (123 µl, 40% in water, 1.07 mmol) and water (1 ml) were added and the mixture heated to 80°C. A saturated solution of NH4Cl (195 µl) was added and the mixture stirred at 100°C for 4 h. The reaction mixture was then cooled to 0°C, treated with solid NaOH until the pH was basic and extracted two times with EtOAc. The extracts were washed with brine, dried over Na2SO4, filtered and evaporated to afford the crude product (266 mg). Chromatography on silica gel afforded the title compound as a white amorphous powder (66 mg, 20%). [α]_{D} = + 47.2° (c=0.25, MeOH)
MS (LC/MS): 301.4 [M+H].

Following the same procedure, the following compounds can be obtained:

### Example 2.1: (1S,3S)-1-(3-Chloro-phenylethynyl)-3-imidazol-1-yl-cyclohexanol

[α]_{D} = -52.7° (c=0.5, MeOH)
MS (LC/MS): 301.4 [M+H]

### Example 2.2: (±)-(1R,3R)-3-imidazol-1-yl-1-m-tolylethynyl-cyclohexanol

MS (LC/MS): 281.3 [M+H]
TLC Rf: 0.52 (EtOAc/MeOH/Et₃N 70:27:3)

Example 3: (±)-(1S,3S)-1-(3-Chloro-phenylethynyl)-3-(2-methyl-imidazol-1-yl)-cyclohexanol A solution of 1-chloro-3-ethynyl-benzene (410 mg, 3 mmol) in diethylether (15 ml) was cooled to -65°. N-BuLi (1.9 ml, 3 mmol, 1.6 M in hexanes) was added dropwise and the mixture stirred for 30' at -65°. A solution of 3-(2-methyl-imidazol-1-yl)-cyclohexanone (446 mg, 2.5 mmol) in THF (2 ml) was added dropwise and the mixture stirred for 90' at -65°. The temperature was allowed to reach room temperature, and stirred for 3 h. The reaction mixture was partitioned between sat. aq. KHCO3 and EtOAc, the aq. phase extracted with EtOAc, the organic phases dried over Na2SO4, filtered and the solvents evaporated. Preparative thin layer chromatography of part of the crude mixture using EtOAc/EtOH/NH4OH 9:1:0.1 as eluent afforded the racemic transisomer (±)-(1S,3S)-1-(3-Chloro-phenylethynyl)-3-(2-methyl-imidazol-1-yl)-cyclohexanol (20 mg) along with the corresponding cis-isomer (see example 3.1, 5 mg). MS (LC/MS): 315 [M+H]. TLC Rf: 0.29 (EtOAc/EtOH/NH4OH 9:1:0.1).

The starting material was prepared as described hereafter:
3-(2-Methyl-imidazol-1-yl)-cyclohexanone: A mixture of cyclohex-2-enone (5.14 g, 53.5 mmol), 2-methyl-1H-imidazole (4.39 g, 53.5 mmol) and bismuthnitrate pentahydrate (3.93 g, 8 mmol) was stirred at room temperature. After 1 h, DCM (4 ml) was added and stirring continued for 15 h. The mixture was filtered, partitioned between EtOAc and sat. aq. NaHCO3, the aq. phase extracted with EtOAc, the combined organic extracts dried over Na2SO4, filtered and the solvents evaporated. The crude residue was dissolved in cyclohexane/EtOAc 1:1, silica gel (10 g) was added and the mixture filtered. The silica gel on the filter was washed first with EtOAc and then with EtOAc/MeOH 4:1. The EtOAc/MeOH 4:1 washings were collected and evaporated to afford the title compound (1.5 g, 16 %) of sufficient purity for subsequent steps.

Following the same procedure, the following compounds can be obtained:

### Example 3.1: (±)-(1S,3R)-1-(3-Chloro-phenylethynyl)-3-(2-methyl-imidazol-1-yl)-cyclohexanol

MS (LC/MS): 315 [M+H]
TLC Rf: 0.25 (EtOAc/EtOH/NH4OH 9:1:0.1)

### Example 3.2: (±)-(1S,3S)-1-(3-Chloro-phenylethynyl)-3-(4-methyl-imidazol-1-yl)-cyclohexanol

MS (LC/MS): 315 [M+H]
TLC Rf: 0.32 (EtOAc/EtOH/NH4OH 9:1:0.1)

### Example 3.3: (±)-(1S,3R)-1-(3-Chloro-phenylethynyl)-3-(4-methyl-imidazol-1-yl)-cyclohexanol

MS (LC/MS): 315 [M+H]
TLC Rf: 0.21 (EtOAc/EtOH/NH4OH 9:1:0.1)

### Example 3.4: (±)-(1S,3S)-1-(3-Chloro-phenylethynyl)-3-(2,4-dimethyl-imidazol-1-yl)-cyclohexanol

MS (LC/MS): 329 [M+H]
TLC Rf: 0.37 (EtOAc/EtOH/NH4OH 9:1:0.1)

### Example 3.5: (±)-(1S,3R)-1-(3-Chloro-phenylethynyl)-3-(2,4-dimethyl-imidazol-1-yl)-cyclohexanol

MS (LC/MS): 329 [M+H]
TLC Rf: 0.27 (EtOAc/EtOH/NH4OH 9:1:0.1)

### Example 3.6: (±)-(1S,3S)-1-(3-Chloro-phenylethynyl)-3-(4-phenyl-imidazol-1-yl)-cyclohexanol

MS (LC/MS): 377 [M+H]
TLC Rf: 0.44 (EtOAc/EtOH/NH4OH 9:1:0.1)

### Example 3.7: (±)-(1S,3S)-1-(3-Chloro-phenylethynyl)-3-(2-isopropyl-imidazol-1-yl)-cyclohexanol

MS (LC/MS): 343 [M+H]
TLC Rf: 0.29 (EtOAc/EtOH/NH4OH 9:1:0.1)

### Example 3.8: (±)-(1S,3S)-3-Benzoimidazol-1-yl-1-(3-chloro-phenylethynyl)-cyclohexanol

MS (LC/MS): 351 [M+H]
TLC Rf: 0.38 (EtOAc/EtOH/NH4OH 9:1:0.1)

### Example 3.9: (±)-(1S,3S)-1-(3-Chloro-phenylethynyl)-3-[1,2,4]triazol-1-yl-cyclohexanol

MS (LC/MS): 302 [M+H]
TLC Rf: 0.36 (EtOAc/EtOH/NH4OH 9:1:0.1)

## Claims

1. A compound of formula (I) 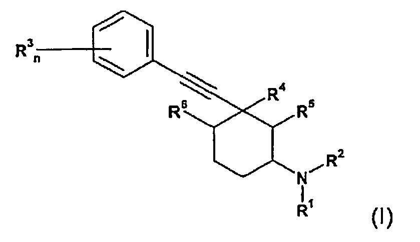 wherein
R¹ represents hydrogen or C₁-C₄ alkyl and
R² represents an unsubstituted or substituted heterocycle or
R¹ represents hydrogen or C₁-C₄ alkyl and
R² represents aryl or substituted aryl or
R¹ and R² together with the nitrogen atom form an unsubstituted or substituted heterocycle;
R³ represents (C₁₋₄)alkyl, (C₁₋₄)alkoxy, trifluoromethyl, halogen, cyano, nitro, -CHO,-COO(C₁₋₄)alkyl, -CO(C₁₋₄)alkyl;
n represents 0, 1, 2, 3, 4 or 5;
R⁴ represents OH and
R⁵ and R⁶ represent H or C₁-C₄ alkyl or
R⁴ and R⁵ form a bond and
R⁶ represent H or C₁-C₄ alkyl or
R⁴ and R⁶ form a bond and
R⁵ represent H or C₁-C₄ alkyl;
in free base or acid addition salt form.

2. A compound of formula (I') 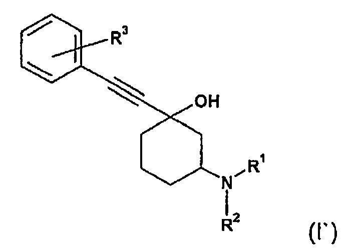 wherein R¹, R², R³ are as defined in claim 1.

3. A process for the preparation of a compound of formula (I) as defined in claim 1, or a salt thereof, which comprises the step of
a)for the production of a compound of formula (I) wherein i) R⁴ represents hydroxy, R¹ represents hydrogen or C₁-C₄ alkyl and R² represents an unsubstituted or substituted heterocycle or ii) R¹ represents hydrogen or C₁-C₄ alkyl and R² represents aryl or substituted aryl, by reductive amination of a compound of formula (II) 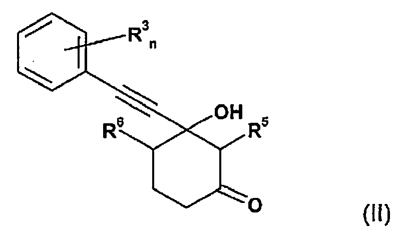 wherein R⁶, R⁵, R³, n are as defined above, with a compound of formula (III) 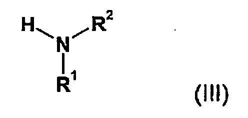 wherein R¹ and R² are as defined above, or
b) for the production of a compound of formula (I) wherein R⁴ represents hydroxy, R¹ and R² together with the nitrogen atom form an unsubstituted or substituted heterocycle, by cyclocondensation of a compound of formula (IV) 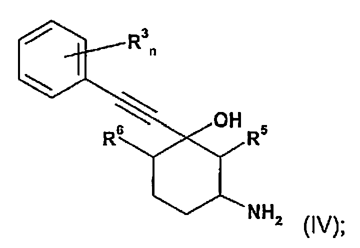 or
c) for the production of a compound of formula (I) wherein R⁴ represents hydroxy, R¹ and R² together with the nitrogen atom form an unsubstituted or substituted heterocycle, by reductive alkylation of a compound of formula (V) 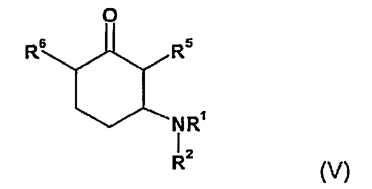 wherein R⁶, R⁵, R², R¹ are as defined above, with a compound of formula (VI) with a compound of formula (VI) 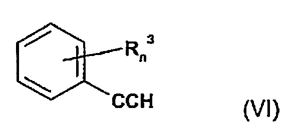 wherein R³ and n are as defined above, or
d) for the production of a compound of formula (I) wherein i) R⁴ and R⁵ form a bond and R⁶ represents hydrogen or C₁-C₄ alkyl or ii) wherein R⁴ and R⁶ form a bond and R⁵ represents hydrogen, by dehydrating a compound of formula (I) wherein R⁴ is hydroxyl R⁵ and R⁶ are hydrogen or C₁-C₄ alkyl;
and recovering the resulting compound of formula (I) in free base or acid addition salt form.

4. A compound of claim 1 in free base or pharmaceutically acceptable acid addition salt form, for use as a pharmaceutical.

5. A compound of claim 1 in free base or pharmaceutically acceptable acid addition salt form, for use in the prevention, treatment or delay of progression of disorders associated with irregularities of the glutamatergic signal transmission, of the gastro-intestinal and urinary tract and of nervous system disorders mediated full or in part by mGluR5.

6. A pharmaceutical composition comprising a compound of claim 1 in free base or pharmaceutically acceptable acid addition salt form, in association with a pharmaceutical carrier or diluent.

7. The use of a compound of claim 1 in free base or pharmaceutically acceptable acid addition salt form, for the manufacture of a pharmaceutical composition designed for the prevention, treatment or delay of progression of disorders associated with irregularities of the glutamatergic signal transmission, of the gastro-intestinal and urinary tract and of nervous system disorders mediated full or in part by mGluR5.

## Patentansprüche

1. Verbindung der Formel (I) 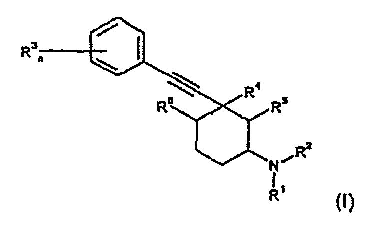 worin
R¹ für Wasserstoff oder C₁-C₄-Alkyl steht und
R² für einen unsubstituierten oder substituierten Heterocyclus steht oder
R¹ für Wasserstoff oder C₁-C₄-Alkyl steht und
R² für Aryl oder substituiertes Aryl steht oder
R¹ und R² gemeinsam mit dem Stickstoffatom einen unsubstituierten oder substituierten Heterocyclus bilden;
R³ für C₁₋₄-Alkyl, C₁₋₄-Alkoxy, Trifluormethyl, Halogen, Cyano, Nitro, -CHO, -COO-C₁₋₄-Alkyl oder-CO-C₁₋₄-Alkyl steht;
n für 0, 1, 2, 3, 4 oder 5 steht;
R⁴ für OH steht und
R⁵ und R⁶ für H oder C₁-C₄-Alkyl stehen oder
R⁴ und R⁵ eine Bindung bilden und
R⁶ für H oder C₁-C₄-Alkyl steht oder
R⁴ und R⁶ eine Bindung bilden und
R⁵ für H oder C₁-C₄-Alkyl steht;
in Form der freien Base oder eines Säureadditionssalzes.

2. Verbindung der Formel (I') 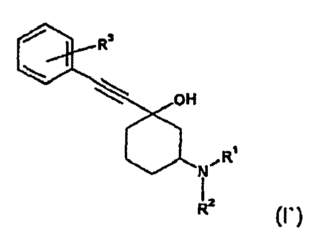 worin R¹, R² und R³ die in Anspruch 1 angegebene Bedeutung besitzen.

3. Verfahren zur Herstellung einer Verbindung der Formel (I) gemäß Anspruch 1 oder eines Salzes davon, bei dem man
a) zur Herstellung einer Verbindung der Formel (I), worin i) R⁴ für Hydroxy steht, R¹ für Wasserstoff oder C₁-C₄-Alkyl steht und R² für einen unsubstituierten oder substituierten Heterocyclus steht oder ii) R¹ für Wasserstoff oder C₁-C₄-Alkyl steht und R² für Aryl oder substituiertes Aryl steht, eine Verbindung der Formel (II) 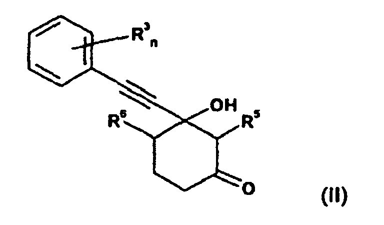 worin R⁶, R⁵, R³ und n die oben angegebene Bedeutung besitzen, mit einer Verbindung der Formel (III) 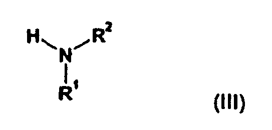 worin R¹ und R² die oben angegebene Bedeutung besitzen, reduktiv aminiert, oder
b) zur Herstellung einer Verbindung der Formel (I), worin R⁴ für Hydroxy steht und R¹ und R² gemeinsam mit dem Stickstoffatom einen unsubstituierten oder substituierten Heterocyclus bilden, eine Verbindung der Formel (IV) 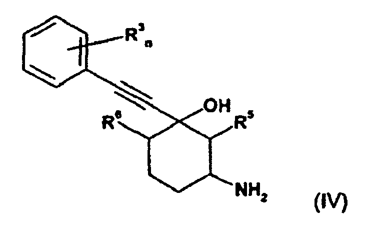 cyclokondensiert, oder
c) zur Herstellung einer Verbindung der Formel (I), worin R⁴ für Hydroxy steht und R¹ und R² gemeinsam mit dem Stickstoffatom einen unsubstituierten oder substituierten Heterocyclus bilden, eine Verbindung (V) 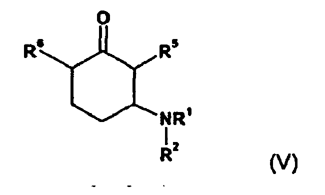 worin R⁶, R⁵, R² und R¹ die oben angegebene Bedeutung besitzen, mit einer Verbindung der Formel (VI) 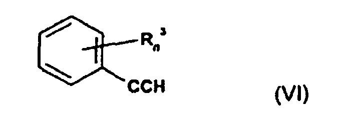 worin R³ und n die oben angegebene Bedeutung besitzen, reduktiv alkyliert, oder
d) zur Herstellung einer Verbindung der Formel (I), worin i) R⁴ und R⁵ eine Bindung bilden und R⁶ für Wasserstoff oder C₁-C₄-Alkyl steht oder ii) worin R⁴ und R⁶ eine Bindung bilden und R⁵ für Wasserstoff steht, eine Verbindung der Formel (I), worin R⁴ für Hydroxy steht und R⁵ und R⁶ für Wasserstoff oder C₁-C₄-Alkyl stehen, dehydriert;
und die erhaltene Verbindung der Formel (I) in Form der freien Base oder eines Säureadditionssalzes gewinnt.

4. Verbindung nach Anspruch 1 in Form der freien Base oder eines pharmazeutisch annehmbaren Säureadditionssalzes zur Verwendung als Pharmazeutikum.

5. Verbindung nach Anspruch 1 in Form der freien Base oder eines pharmazeutisch annehmbaren Säureadditionssalzes zur Verwendung bei der Prävention, Behandlung oder Verzögerung des Fortschreitens von Störungen, die mit Unregelmäßigkeiten der glutamatergen Signalübertragung assoziiert sind, des Magen-Darm- und Harntrakts und von Störungen des Nervensystems, die ganz oder teilweise durch mGluR5 vermittelt werden.

6. Pharmazeutische Zusammensetzung, die eine Verbindung nach Anspruch 1 in Form der freien Base oder eines pharmazeutisch annehmbaren Säureadditionssalzes zusammen mit einem pharmazeutischen Träger oder Verdünnungsmittel enthält.

7. Verwendung einer Verbindung nach Anspruch 1 in Form einer freien Base oder eines pharmazeutisch annehmbaren Säureadditionssalzes zur Herstellung einer pharmazeutischen Zusammensetzung zur Prävention, Behandlung oder Verzögerung des Fortschreitens von Störungen, die mit Unregelmäßigkeiten der glutamatergen Signalübertragung assoziiert sind, des Magen-Darm- und Harntrakts und von Störungen des Nervensystems, die ganz oder teilweise durch mGluR5 vermittelt werden.

## Revendications

1. Composé de formule (I) 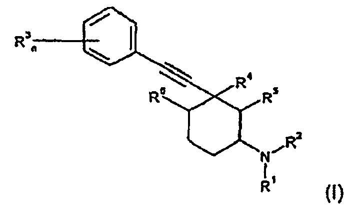 dans laquelle
R¹ représente hydrogène ou C₁-C₄ alkyle et
R² représente un hétérocycle non substitué ou substitué ou
R¹ représente hydrogène ou C₁-C₄ alkyle et
R² représente aryle ou aryle substitué ou
R¹ et R² ensemble avec l'atome d'azote forment un hétérocycle non substitué ou substitué;
R³ représente (C₁₋₄) alkyle, (C₁₋₄)alcoxy, trifluorométhyle, halogène, cyano, nitro, -CHO, -COO(C₁₋₄) alkyle, -CO(C₁₋₄)alkyle ;
n représente 0, 1, 2, 3, 4 ou 5 ;
R⁴ représente OH et
R⁵ et R⁶ représentent H ou C₁-C₄ alkyle ou
R⁴ et R⁵ forment une liaison et
R⁶ représente H ou C₁-C₄ alkyle ou
R⁴ et R⁶ forment une liaison et
R⁵ représente H ou C₁-C₄ alkyle ;
sous forme de base libre ou de sel d'addition d'acide.

2. Composé de formule (I') 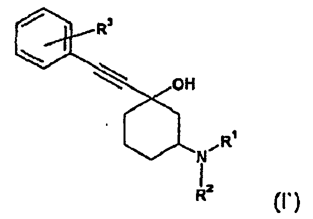 dans laquelle R¹, R², R³ sont tels que définis dans la revendication 1.

3. Procédé pour la préparation d'un composé de formule (I) tel que défini dans la revendication 1, ou d'un sel de celui-ci, qui comprend l'étape suivante :
a) pour la production d'un composé de formule (I) dans laquelle i) R⁴ représente hydroxy, R¹ représente hydrogène ou C₁-C₄ alkyle et R² représente un hétérocycle non substitué ou substitué ou ii) R¹ représente hydrogène ou C₁-C₄ alkyle et R² représente aryle ou aryle substitué, l'amination réductrice d'un composé de formule (II) 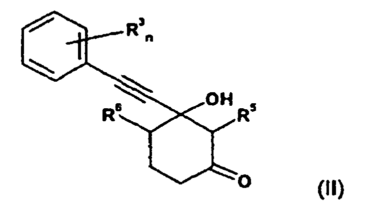 dans laquelle R⁶, R⁵, R³, n sont tels que définis ci-dessus, avec un composé de formule (III) 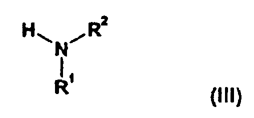 dans laquelle R¹ et R² sont tels que définis ci-dessus, ou
b) pour la production d'un composé de formule (I) dans laquelle R⁴ représente hydroxy, R¹ et R² ensemble avec l'atome d'azote forment un hétérocycle non substitué ou substitué, la cyclocondensation d'un composé de formule (IV) 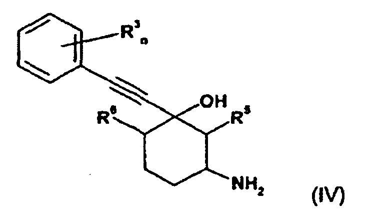 ou
c) pour la production d'un composé de formule (I) dans laquelle R⁴ représente hydroxy, R¹ et R² ensemble avec l'atome d'azote forment un hétérocycle non substitué ou substitué, l'alkylation réductrice d'un composé de formule (V) 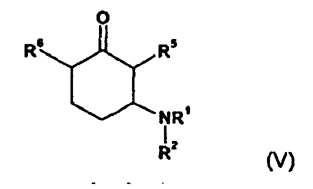 dans laquelle R⁶, R⁵, R², R¹ sont tels que définis ci-dessus, avec un composé de formule (VI) 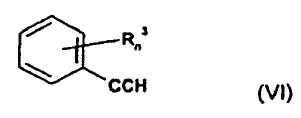 dans laquelle R³ et n sont tels que définis ci-dessus, ou
d) pour la production d'un composé de formule (I) dans laquelle i) R⁴ et R⁵ forment une liaison et R⁶ représente hydrogène ou C₁-C₄ alkyle ou ii) dans laquelle R⁴ et R⁶ forment une liaison et R⁵ représente hydrogène, la déshydratation d'un composé de formule (I) dans laquelle R⁴ est hydroxy, R⁵ et R⁶ sont hydrogène ou C₁-C₄ alkyle,
et la récupération du composé résultant de formule (I) sous forme de base libre ou de sel d'addition d'acide.

4. Composé selon la revendication 1, sous forme de base libre ou de sel d'addition d'acide pharmaceutiquement acceptable, destiné à être utilisé comme produit pharmaceutique.

5. Composé selon la revendication 1, sous forme de base libre ou de sel d'addition d'acide pharmaceutiquement acceptable, destiné à être utilisé pour prévenir, traiter ou retarder la progression de troubles associés à des irrégularités de la transmission du signal glutamatergique, des troubles de la voie gastro-intestinale et urinaire et de troubles du système nerveux médiés totalement ou en partie par le mGluR5.

6. Composition pharmaceutique comprenant un composé selon la revendication 1 sous forme de base libre ou de sel d'addition d'acide pharmaceutiquement acceptable, en association avec un support ou un diluant pharmaceutique.

7. Utilisation d'un composé selon la revendication 1 sous forme de base libre ou de sel d'addition d'acide pharmaceutiquement acceptable, pour la fabrication d'une composition pharmaceutique conçue pour prévenir, traiter ou retarder la progression de troubles associés à des irrégularités de la transmission du signal glutamatergique, des troubles de la voie gastro-intestinale et urinaire et de troubles du système nerveux médiés totalement ou partiellement par le mGluR5.
